# EUROPEAN PATENT SPECIFICATION

(11) **EP 3 227 320 B1**
(45) Date of publication and mention of the grant of the patent: **03.07.2019**
(21) Application number: 15865723.9
(22) Date of filing: 02.12.2015
(51) Int. Cl.: A61K 38/00, C07K 14/50

(54) **MODIFIED FIBROBLAST GROWTH FACTOR 1 (FGF-1) POLYPEPTIDES WITH INCREASED BINDING AFFINITY FOR HEPARIN AND ASSOCIATED METHODS**
POLYPEPTIDE MIT MODIFIZIERTEM FIBROBLASTEN-WACHSTUMSFAKTOR 1 (FGF-1) MIT ERHÖHTER BINDUNGSAFFINITÄT FÜR HEPARIN UND ZUGEHÖRIGES VERFAHREN
POLYPEPTIDES DE FACTEUR DE CROISSANCE DES FIBROBLASTES 1 (FGF -1) MODIFIÉ PRÉSENTANT UNE AFFINITÉ DE LIAISON ACCRUE POUR L'HÉPARINE ET PROCÉDÉS ASSOCIÉS

(30) Priority: 03.12.2014 US 201462086788 P
(43) Date of publication of application: 11.10.2017
(73) Proprietor: Florida State University Research Foundation, Inc., Tallahassee, Florida 32310 (US)
(72) Inventor: BLABER, Michael, Tallahassee, Florida 32301 (US); XIA, Xue, Tallahasse, Florida 32303 (US)
(74) Representative: J A Kemp
(86) International application number: PCT/US2015/063333
(87) International publication number: WO 2016/089945

(56) References cited:
- WO-A1-2013/120629
- WO-A2-2016/172153
- US-A1- 2014 045 751
- US-A1- 2015 148 293
- SHIREMAN P K ET AL: "The S130K fibroblast growth factor-1 mutant induces heparin-independent proliferation and is resistant to thrombin degradation in fibrin glue", JOURNAL OF VASCULAR SURGERY, C.V. MOSBY CO., ST. LOUIS, MO, US, vol. 31, no. 2, 1 February 2000 (2000-02-01), pages 382-390, XP002350859, ISSN: 0741-5214, DOI: 10.1016/S0741-5214(00)90168-X
- BREWSTER ET AL: "Improving endothelial healing with novel chimeric mitogens", AMERICAN JOURNAL OF SUR, PAUL HOEBER, NEW YORK, NY, US, vol. 192, no. 5, 1 November 2006 (2006-11-01), pages 589-593, XP005721385, ISSN: 0002-9610, DOI: 10.1016/J.AMJSURG.2006.08.005
- BREWSTER L P ET AL: "Heparin-independent mitogenicity in an endothelial and smooth muscle cell chimeric growth factor (S130K-HBGAM)", AMERICAN JOURNAL OF SUR, PAUL HOEBER, NEW YORK, NY, US, vol. 188, no. 5, 1 November 2004 (2004-11-01), pages 575-579, XP004641818, ISSN: 0002-9610, DOI: 10.1016/J.AMJSURG.2004.07.012
- ANGELES CANALES ET AL: "Solution NMR structure of a human FGF-1 monomer, activated by a hexasaccharide heparin-analogue", FEBS JOURNAL, vol. 273, no. 20, 1 October 2006 (2006-10-01), pages 4716-4727, XP55452455, GB ISSN: 1742-464X, DOI: 10.1111/j.1742-4658.2006.05474.x
- KLINGENBERG O ET AL: "Effects of mutations of a phosphorylation site in an exposed loop in acidic fibroblast growth factor", JOURNAL OF BIOLOGICAL CHEMISTRY, AMERICAN SOCIETY FOR BIOCHEMISTRY AND MOLECULAR BIOLOGY, US, vol. 274, no. 25, 18 June 1999 (1999-06-18), pages 18081-18086, XP002627206, ISSN: 0021-9258, DOI: 10.1074/JBC.274.25.18081
- CANALES, A. ET AL.: 'Solution NMR structure of a human FGF-1 monomer, activated by a hexasaccharide heparin-analogue.' FEBS JOURNAL vol. 273, no. 20, 01 October 2006, pages 4716 - 4727, XP055452455 DOI: 10.1111/J.1742-4658.2006.05474.X

### SEQUENCE LISTING

<110> The Florida State University Research Foundation, Inc.
<120> MODIFIED FIBROBLAST GROWTH FACTOR 1 (FGF-1) POLYPEPTIDES WITH INCREASED BINDING AFFINITY FOR HEPARIN
<130> 232109-00950
<160> 4
<170> PatentIn version 3.5
<210> 1
   <211> 140
   <212> PRT
   <213> Homo sapiens
<400> 1
   Phe Asn Leu Pro Pro Gly Asn Tyr Lys Lys Pro Lys Leu Leu Tyr Cys
   1               5                   10                  15
   Ser Asn Gly Gly His Phe Leu Arg Ile Leu Pro Asp Gly Thr Val Asp
               20                  25                  30
   Gly Thr Arg Asp Arg Ser Asp Gln His Ile Gln Leu Gln Leu Ser Ala
           35                  40                  45
   Glu Ser Val Gly Glu Val Tyr Ile Lys Ser Thr Glu Thr Gly Gln Tyr
       50                  55                  60
   Leu Ala Met Asp Thr Asp Gly Leu Leu Tyr Gly Ser Gln Thr Pro Asn
   65                  70                  75                  80
   Glu Glu Cys Leu Phe Leu Glu Arg Leu Glu Glu Asn His Tyr Asn Thr
                   85                  90                  95
   Tyr Ile Ser Lys Lys His Ala Glu Lys Asn Trp Phe Val Gly Leu Lys
               100                 105                 110
   Lys Asn Gly Ser Cys Lys Arg Gly Pro Arg Thr His Tyr Gly Gln Lys
           115                 120                 125
   Ala Ile Leu Phe Leu Pro Leu Pro Val Ser Ser Asp
       130                 135                 140
<210> 2
   <211> 140
   <212> PRT
   <213> Artificial Sequence
<220>
   <223> Synthetic Peptide
<400> 2
   Phe Asn Leu Pro Pro Gly Asn Tyr Lys Lys Pro Lys Leu Leu Tyr Cys
   1               5                   10                  15
   Ser Asn Gly Gly His Phe Leu Arg Ile Leu Pro Asp Gly Thr Val Asp
               20                  25                  30
   Gly Thr Arg Asp Arg Ser Asp Gln His Ile Gln Leu Gln Leu Ser Ala
           35                  40                  45
   Glu Ser Val Gly Glu Val Tyr Ile Lys Ser Thr Glu Thr Gly Gln Tyr
       50                  55                  60
   Leu Ala Met Asp Thr Asp Gly Leu Leu Tyr Gly Ser Gln Thr Pro Asn
   65                  70                  75                  80
   Glu Glu Cys Leu Phe Leu Glu Arg Leu Glu Glu Asn His Tyr Asn Thr
                   85                  90                  95
   Tyr Ile Ser Lys Lys His Ala Glu Lys Asn Trp Phe Val Gly Leu Lys
               100                 105                 110
   Lys Asn Gly Arg Cys Lys Arg Gly Pro Arg Thr His Tyr Gly Gln Lys
           115                 120                 125
   Ala Ile Leu Phe Leu Pro Leu Pro Val Ser Ser Asp
       130                 135                 140
<210> 3
   <211> 140
   <212> PRT
   <213> Artificial Sequence
<220>
   <223> Synthetic Peptide
<400> 3
   Phe Asn Leu Pro Pro Gly Asn Tyr Lys Lys Pro Lys Leu Leu Tyr Cys
   1               5                   10                  15
   Ser Asn Gly Gly His Phe Leu Arg Ile Leu Pro Asp Gly Thr Val Asp
               20                  25                  30
   Gly Thr Arg Asp Arg Ser Asp Gln His Ile Gln Leu Gln Leu Ser Ala
           35                  40                  45
   Glu Ser Val Gly Glu Val Tyr Ile Lys Ser Thr Glu Thr Gly Gln Tyr
       50                  55                  60
   Leu Ala Met Asp Thr Asp Gly Leu Leu Tyr Gly Ser Gln Thr Pro Asn
   65                  70                  75                  80
   Glu Glu Cys Leu Phe Leu Glu Arg Leu Glu Glu Asn His Tyr Asn Thr
                   85                  90                  95
   Tyr Ile Ser Lys Lys His Ala Glu Lys Asn Trp Phe Val Gly Leu Lys
               100                 105                 110
   Lys Asn Gly Lys Cys Lys Arg Gly Pro Arg Thr His Tyr Gly Gln Lys
           115                 120                 125
   Ala Ile Leu Phe Leu Pro Leu Pro Val Ser Ser Asp
       130                 135                 140
<210> 4
   <211> 155
   <212> PRT
   <213> Homo sapiens
<400> 4
   Met Ala Glu Gly Glu Ile Thr Thr Phe Thr Ala Leu Thr Glu Lys Phe
   1               5                   10                  15
   Asn Leu Pro Pro Gly Asn Tyr Lys Lys Pro Lys Leu Leu Tyr Cys Ser
               20                  25                  30
   Asn Gly Gly His Phe Leu Arg Ile Leu Pro Asp Gly Thr Val Asp Gly
           35                  40                  45
   Thr Arg Asp Arg Ser Asp Gln His Ile Gln Leu Gln Leu Ser Ala Glu
       50                  55                  60
   Ser Val Gly Glu Val Tyr Ile Lys Ser Thr Glu Thr Gly Gln Tyr Leu
   65                  70                  75                  80
   Ala Met Asp Thr Asp Gly Leu Leu Tyr Gly Ser Gln Thr Pro Asn Glu
                   85                  90                  95
   Glu Cys Leu Phe Leu Glu Arg Leu Glu Glu Asn His Tyr Asn Thr Tyr
               100                 105                 110
   Ile Ser Lys Lys His Ala Glu Lys Asn Trp Phe Val Gly Leu Lys Lys
           115                 120                 125
   Asn Gly Ser Cys Lys Arg Gly Pro Arg Thr His Tyr Gly Gln Lys Ala
       130                 135                 140
   Ile Leu Phe Leu Pro Leu Pro Val Ser Ser Asp
   145                 150                 155

## Description

### Field

This relates to the field of human growth factors and, more particularly, to making growth factors safer for human therapeutic use.

### Background

Insufficient blood flow is the source of a number of diseases and other physiological conditions. These include peripheral artery disease and conditions that cause skin lesions such as diabetic ulcers and bed sores.

Insufficient blood flow may be treated with a growth factor because growth factors promote new blood vessel growth and regeneration of tissue. Several growth factors have been evaluated in both pre-clinical and clinical studies. Such growth factors include vascular endothelial cell growth factor (VEGF), platelet derived growth factor (PDGF), fibroblast growth factor 1 and 2 (FGF-1 and FGF-2), and keratinocyte growth factor (KGF).

A drawback to using one or more of these growth factors is that they have poor pharmacokinetic profiles. They have a fast clearance rate from the body after administration, resulting in the need for frequent and repetitive dosing. They also distribute throughout the body after administration, which may cause undesirable side-effects.

An important safety concern for using growth factors is to minimize their systemic distribution to prevent negative off-target effects. When administered, growth factors can diffuse into the circulatory system, travel throughout the body, and stimulate the growth of cancer cells that might exist far from the site where the growth factor was administered.

A pharmacokinetic study from 2012 showed that wild-type FGF-1 binds to heparin. According to that study, the pharmacokinetic profile of FGF-1 is predominantly determined by the binding affinity of FGF-1 to heparan sulfate glycosaminoglycan (HSGAG), which is also known as heparan sulfate proteoglycan (HSPG). HSGAG is a sulfated or acetylated polysaccharide located on the surface of different cells and tissues, such as vascular endothelial cells of the liver and kidney. *See*, X. Xia, et al., "Pharmacokinetic properties of 2nd-generation fibroblast growth factor-1 mutants for therapeutic application." PLoS ONE 7(11): e48210 2012.
Shireman *et al.* discloses that the S130K fibroblast growth factor-1 mutant induces heparin-independent proliferation and is resistant to thrombin degradation in fibrin glue.

### Cited Documents

P.K. Shireman, L. Xue, B. Hampton, W.H. Burgess, H.P. Greisler The cysteine-free fibroblast growth factor 1 mutant induces heparin-independent proliferation of endothelial cells and smooth muscle cells, J. Surg. Res., 92 (2000), pp. 255-260

### Brief Summary

It would be advantageous to improve the binding affinity of FGF-1 to heparin to improve the likelihood that FGF-1 will substantially remain at the site of administration rather than distributing throughout the body. The problem of increasing FGF-1's binding affinity to heparin is solved by modifying the polypeptide sequence of FGF-1 to have an increased binding affinity for heparin relative to the binding affinity of wild-type FGF-1 to heparin.

The present invention provides a modified FGF-1 polypeptide having at least 80% amino acid sequence identity to wild-type human FGF-1 having SEQ ID NO: 1, the serine at an amino acid position of the modified FGF-1 polypeptide corresponding to amino acid position 116 of SEQ ID NO: 1 being substituted by an arginine, and wherein the arginine increases the modified FGF-1 polypeptide's binding affinity for heparin relative to the binding affinity of SEQ ID NO:1 to heparin.

In an example of a composition aspect, the composition comprises a modified FGF-1 polypeptide having at least 80% amino acid sequence identity to wild-type human FGF-1 having SEQ ID NO: 1. The serine at an amino acid position of the modified FGF-1 polypeptide corresponding to amino acid position 116 of SEQ ID NO: 1 is substituted by an arginine, wherein the arginine increases the modified FGF-1 polypeptide's binding affinity for heparin relative to the binding affinity of SEQ ID NO: 1 to heparin.

In an example of a first method aspect, the method comprises increasing the binding affinity to heparin of wild- type human FGF-1 having SEQ ID NO: 1 by forming a modified FGF-1 polypeptide having at least 80% amino acid sequence identity to SEQ ID NO: 1. The serine at an amino acid position of the modified FGF-1 polypeptide corresponding to amino acid position 116 of SEQ ID NO: 1 is an arginine, wherein the arginine increases the modified FGF-1 polypeptide's binding affinity for heparin relative to the binding affinity of SEQ ID NO: 1 to heparin.

Another aspect provides a modified peptide for use in a method of treating a physiological condition associated with insufficient blood flow, wherein the method comprises administering to a subject in need thereof a composition comprising a therapeutically effective amount of a modified FGF-1 polypeptide having at least 80% amino acid sequence identity to wild-type human FGF-1 having SEQ ID NO: 1. The serine at an amino acid position of the modified FGF-1 polypeptide corresponding to amino acid position 116 of SEQ ID NO: 1 is substituted by an arginine, wherein the arginine increases the modified FGF-1 polypeptide's binding affinity for heparin relative to the binding affinity of SEQ ID NO: 1 to heparin.

Another aspect provides a modified peptide for use in a method of treating peripheral artery disease, skin lesions, ischemia, hypoxia or avascular necrosis, the method comprising administering to a subject in need thereof a composition comprising a therapeutically effective amount of a modified FGF-1 polypeptide having at least 80% amino acid sequence identity to wild-type human FGF-1 having SEQ ID NO: 1, the serine at an amino acid position of the modified FGF-1 polypeptide corresponding to amino acid position 116 of SEQ ID NO: 1 is substituted by an arginine, and wherein the arginine increases the modified FGF-1 polypeptide's binding affinity for heparin relative to the binding affinity of SEQ ID NO: 1 to heparin.

Another aspect provides a modified peptide for use in a method of treating a wound comprises administering to a human subject having a wound a composition comprising a therapeutically effective amount of a modified FGF-1 polypeptide having at least 80% amino acid sequence identity to wild-type human FGF-1 having SEQ ID NO:1. The serine at an amino acid position of the modified FGF-1 polypeptide corresponding to amino acid position 116 of SEQ ID NO:1 is substituted by an arginine, wherein the arginine increases the modified FGF-1 polypeptide's binding affinity for heparin relative to the binding affinity of SEQ ID NO: 1 to heparin.

In the compositions and methods, the modified FGF-1 polypeptide may comprise SEQ ID NO: 2 or consist of SEQ ID NO: 2.

In the compositions and methods, the modified FGF-1 polypeptide may comprises SEQ ID NO: 3 or consist of SEQ ID NO: 3.

In the compositions and methods, the amino acid that increases the modified FGF-1 polypeptide's binding affinity for heparin is arginine.

Disclosed herein, the amino acid that increases the modified FGF-1 polypeptide's binding affinity for heparin may be lysine.

In the compositions and methods, the modified FGF-1 polypeptide may have at least 90% amino acid sequence identity to wild-type human FGF-1 having SEQ ID NO: 1 and/or it may have at least 99% amino acid sequence identity to wild-type human FGF-1 having SEQ ID NO: 1.

### Brief Description of the Drawings

**FIG.1** is the polypeptide sequence of the 140 amino acid form of wild-type human FGF-1 (SEQ ID NO:1) with the serine (S) at position 116 (Ser116) highlighted.
**FIG.2** is the polypeptide sequence of the 154 amino acid form of wild-type human FGF-1 (SEQ ID NO: 4) with the position corresponding to Ser116 of the 140 amino acid form highlighted. The amino acids in positions 1-15 are shaded because they are omitted from the 140 amino acid form.
**FIG.3** is the polypeptide sequence of a first example of modified FGF-1 (SEQ ID NO: 2) with a box indicating arginine (R) at position 116.
**FIG.4** is the polypeptide sequence of a second example of modified FGF-1 (SEQ ID NO: 3) with a box indicating lysine (K) at position 116.

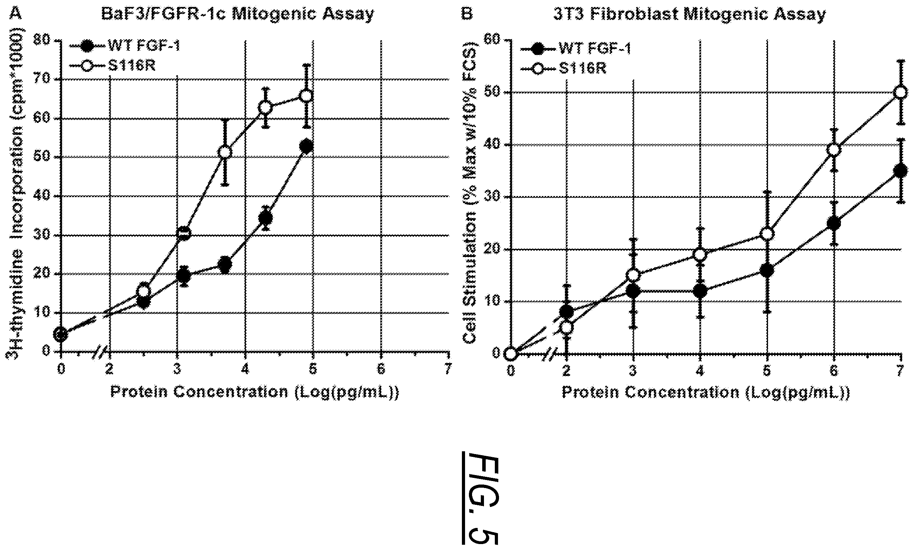

**FIG. 5A** is a graph illustrating the induced cell proliferation of BaF3 cells expressing FGFR-lc by wild-type FGF- 1 and a modified FGF-1 (S116R), quantified by radioactive ³H-thimidine incorporation. The protein concentration (pg/mg) is plotted as loglO scale. The wild-type FGF-1 is represented by filled circles and the modified FGF-1 is represented by open circles.
**FIG. 5B** is a graph illustrating the mitogenic activity of wild-type FGF-1 and S116R with NIH 3T3 fibroblasts as determined from cell counts normalized to the maximum stimulation achieved by 10% FCS controls. The protein concentration (pg/mL) is plotted as log10 scale. The WT FGF-1 is represented by filled circles and the modified FGF-1 (S116R) is represented by open circles.

### Detailed Description

Modifications may be made to wild type human FGF-1 to increase its binding affinity for heparin. Because the modified FGF-1 has a higher binding affinity for heparin than wild-type FGF-1, using modified FGF-1 in a therapeutic treatment can lower the systemic distribution of FGF-1. This means that when modified FGF-1 is administered to a portion of the body, it is more likely to remain at that portion of the body because it is bound to heparin. This increases the mean residence time of FGF-1 at the desired location, thereby improving the efficacy of FGF-1 and decreasing the dosage level or the need to re-administer FGF-1 to achieve efficacy.

The term "binding affinity" refers, for example, to how strong a polypeptide's binding activity is for a particular target such as heparin. There are many ways to quantify the binding affinity of a polypeptide to heparin. Examples include, but are not limited to, thermodynamic techniques such as isothermal titration calorimetry.

The term "heparin" refers, for example, to heparin, heparan sulfate, heparan sulfate proteoglycan, and heparan sulfate glycosaminoglycan.

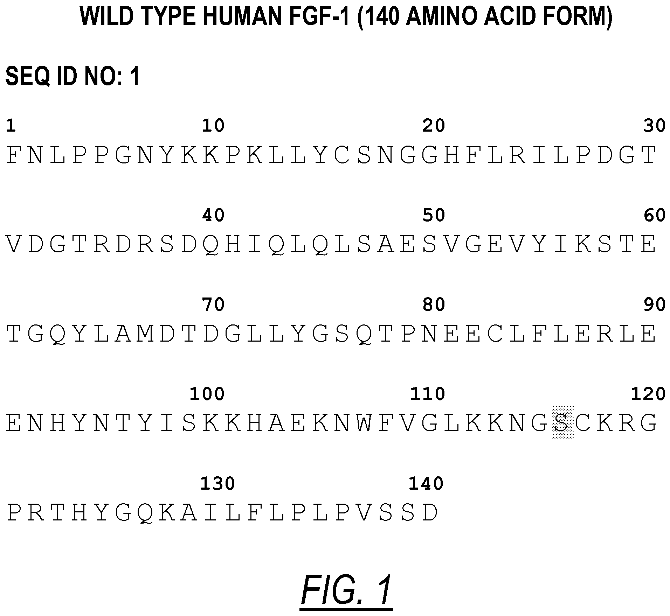

**FIG. 1** and SEQ ID NO: 1 are the polypeptide sequence of the 140 amino acid form of wild-type human FGF-1. The numbering scheme used throughout this disclosure is based on the 140 amino acid form, beginning with F (Phe) at position 1 and ending with D (Asp) at position 140.

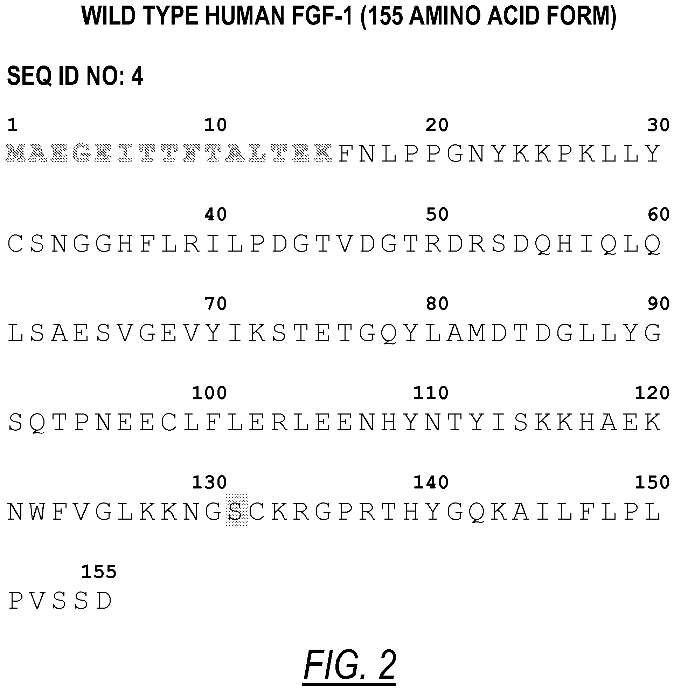

Other forms of FGF-1, such as the 155 amino acid form, include SEQ ID NO: 1. The 140 amino acid form is produced by proteolysis of the 155 amino acid form. The 155 amino acid form is in **FIG. 2** and SEQ ID NO: 4. Positions 1-15 of the 155 amino acid form are shaded because they are omitted from the 140 amino acid form. The modification at Ser116 of 155 amino acid form, based on the numbering scheme of the 140 amino acid form, will also increase the binding affinity for heparin. The position corresponding to Ser116 of the 140 amino acid form is Ser131, which is highlighted.

There is also a 154 amino acid form that differs from the 155 amino acid form by deleting methionine at position 1 of the 155 amino acid form. When FGF-1 is purified from natural tissue, the 154 and 140 amino acid forms are present. The methionine at position 1 is removed. The numbering scheme for the 154 amino acid form is simply shifted by 1 to account for the removed methionine.

As shown in **FIG. 1**, the amino acid at the 116 position of wild-type human FGF-1 is S (Ser or serine). This position of wild type human FGF-1 is referred to as Ser116. Substituting other amino acids for Ser116 forms a modified FGF-1 having an increased binding affinity for heparin relative to wild type human FGF-1.

Examples of compositions that include a modified FGF-1 polypeptide with increased binding affinity to heparin will now described.

The composition generally incudes a modified FGF-1 polypeptide having at least 80% amino acid sequence identity to wild-type human FGF-1 having SEQ ID NO: 1. The serine at an amino acid position of the modified FGF-1 polypeptide corresponding to amino acid position 116 of SEQ ID NO: 1 is substituted by an amino acid that increases the modified FGF-1 polypeptide's binding affinity for heparin relative to the binding affinity of SEQ ID NO: 1 to heparin.

Examples of amino acids that increase the modified FGF-1 polypeptide's binding affinity for heparin relative to the binding affinity of SEQ ID NO: 1 to heparin include arginine (Arg) and lysine (Lys).

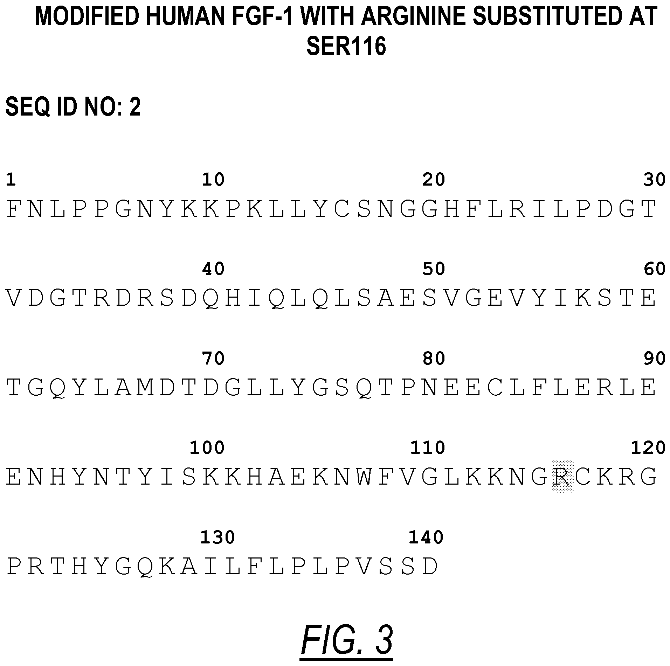

A particular example of the modified FGF-1 polypeptide is in **FIG. 3** and SEQ ID NO: 2. In this example, Ser116 is substituted by Arg (R). SEQ ID NO: 2 is identical to SEQ ID NO: 1 except for this substitution.

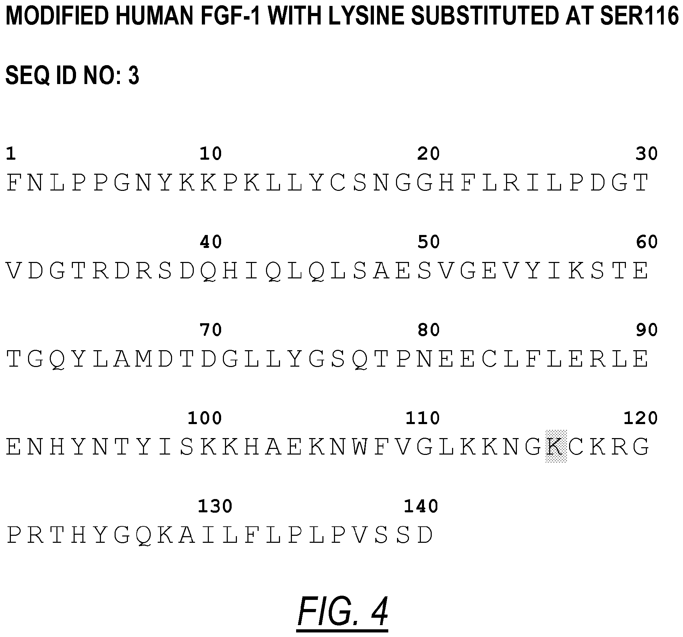

Another particular example of the modified FGF-1 polypeptide is in **FIG. 4** and SEQ ID NO: 3. In this example, Ser116 is substituted by Lys (K). SEQ ID NO: 3 is identical to SEQ ID NO: 1 except for this substitution.

The modified FGF-1 polypeptide does not need not have such close amino acid sequence identity with SEQ ID NO: 1 as SEQ ID NOS: 2 and 3 have, so long as the mitogenic function of SEQ ID NO: 1 is preserved in the modified FGF-1.

Having at least 80% amino acid sequence identity to wild-type human FGF-1 means that at least 80% of the amino acid residues of multiple polypeptides being compared to each other have the same amino acid at the corresponding position on the other polypeptide. For example, a polypeptide that has least 80% amino acid sequence identity to another polypeptide sequence has at least 80% of its amino acid residues at the corresponding position on the other polypeptide.

In another example, the modified FGF-1 polypeptide has at least 90% amino acid sequence identity to wild-type human FGF-1. This means that at least 90% of the amino acid residues of multiple polypeptides being compared to each other have the same amino acid at the corresponding position on the other polypeptide. For example, a polypeptide that has least 90% amino acid sequence identity to another polypeptide sequence has at least 90% of its amino acid residues at the corresponding position on the other polypeptide.

In an additional example, the modified FGF-1 polypeptide has at least 99% amino acid sequence identity to wild-type human FGF-1. This means that at least 99% of the amino acid residues of multiple polypeptides being compared to each other have the same amino acid at the corresponding position on the other polypeptide. For example, a polypeptide that has least 99% amino acid sequence identity to another polypeptide sequence has at least 99% of its amino acid residues at the corresponding position on the other polypeptide.

In these examples of the modified FGF-1 polypeptide, an amino acid residue at a particular position on one polypeptide does not have to be at exactly the same position on another polypeptide to qualify as being at a "corresponding" position. Different polypeptides may have different amino acid positions and numbering schemes, depending on how similar the polypeptides are. Different polypeptides may have different lengths, insertions, mutations, substitutions, and/or deletions, which would offset the amino acid numbering scheme. Amino acid positions on different polypeptides may correspond to each other if the amino acid positions are similar when compared to their tertiary structures. A skilled artisan can determine whether amino acid positions on different polypeptides correspond to each other based on their primary and/or tertiary structures.

The modification to the FGF-1 polypeptide at Ser116 may be a gain-of-function mutation that enhances the mitogenic activity of the modified FGF-1 polypeptide relative to the mitogenic activity of the wild-type human FGF-1 polypeptide. This gain-of-function may or may not be associated with increased heparin binding affinity.

The modified FGF-1 polypeptide may include the modification at Ser116 or a corresponding position in combination with one or more additional modifications. For example, other amino acids may be substituted to stabilize the modified FGF-1 and/or to remove the free cysteine residues.

The modified FGF-1 polypeptide may be an active ingredient in a pharmaceutical composition. In such a case, the modified FGF-1 polypeptide may be blended with one or more pharmaceutically acceptable excipients useful for making the composition into a pharmaceutically acceptable dosage form.

Exemplary excipients include, but are not limited to, carriers, diluents, disintegrants, emulsifiers, solvents, processing aids, buffering agents, colorants, flavorings, solvents, coating agents, binders, carriers, glidants, lubricants, granulating agents, gelling agents, polishing agents, suspending agent, sweetening agent, anti-adherents, preservatives, emulsifiers, antioxidants, plasticizers, surfactants, viscosity agents, enteric agents, wetting agents, thickening agents, stabilizing agents, solubilizing agents, bioadhesives, film forming agents, emollients, dissolution enhancers, dispersing agents, or combinations thereof.

Since the pharmaceutical composition includes a polypeptide, the pharmaceutical composition may include at least one substance that substantially inhibits protein degradation.

The pharmaceutical composition may include one or more additional growth factors and/or angiogenic compounds, such as, for example, VEGF, PDGF, KGF, other wild-type and/or mutant FGF proteins, and vasodilators.

In the pharmaceutical composition, the modified FGF-1 polypeptide is present in a therapeutically effective amount. A therapeutically effective amount is an amount effective to achieve a desired therapeutic benefit, such as an amount effective to prevent, alleviate, ameliorate, or treat the underlying causes and/or symptoms of the physiological condition being treated.

For some uses of the pharmaceutical composition, a therapeutically effective amount is an amount effective to increase blood flow, stimulate angiogenesis, and/or vascularization within or to a particular tissue or region of the body of a treatment subject. The degree to which blood flow, angiogenesis, and/or vascularization is increased within a region of the body may be determined by conventional pathological or clinical techniques, such as imaging techniques using a contrast dye to detect vasculature or the reduction of symptoms associated with an underlying disorder.

For some uses of the pharmaceutical composition, a therapeutically effective amount may be an amount effective to improve the quality and/or rate of healing or repair of a damaged tissue or wound. The improvement is quantifiable according to conventional clinical practices.

When the pharmaceutical composition is administered to cardiac tissue, a therapeutically effective amount may be an amount effective to reduce clinical symptoms of peripheral artery disease, such as reduction in angina, breathlessness, leg swelling, heart or respiratory rates, edema, fatigue, or weakness, or to reduce the risk of a myocardial infarction.

In humans, a therapeutically effective amount range is often 1-1,000 mg/day, including 1-25 mg/day, 25-50 mg/day, 50-75 mg/day, 75-100 mg/day, 100-150 mg/day, 150-200 mg/day, 200-250 mg/day, 250-300 mg/day, 300-350 mg/day, 350-400 mg/day, 400-450 mg/day, 450-500 mg/day, 500-550 mg/day, 550-600 mg/day, 600-650 mg/day, 650-700 mg/day, 700-750 mg/day, 750-800 mg/day, 800-850 mg/day, 850-900 mg/day, 900-950 mg/day, 950-1,000 mg/day. Higher doses (1,000-3,000 mg/day) might also be effective. The weight in mg is often calibrated to the body weight of the subject in kg, thus these example doses may also be written in terms of mg/kg of body weight per day.

In practice, the therapeutically effective amount may vary depending on numerous factors associated with the treatment subject, including age, weight, height, severity of the disorder, administration technique, and other factors. The therapeutically effective amount of the modified FGF-1 polypeptide administered to a given subject may be determined by medical personnel taking into account the relevant circumstances.

The therapeutically effective amount may be determined or predicted from empirical evidence. Specific dosages may vary according to numerous factors and may be initially determined on the basis of in vitro, cell culture, and/or animal in vivo studies. Dosages or concentrations tested in vitro for modified FGF-1 polypeptides may provide useful guidance in determining therapeutically effective and appropriate amounts for administration.

The pharmaceutical composition may be administered as a single dose or as part of a dosage regimen. For a dosage regimen, the therapeutically effective amount is adjustable dose to dose to provide a desired therapeutic response.

Multiple doses may be administered at a predetermined time interval and subsequent doses may be proportionally reduced, depending on the situation. By administering the pharmaceutical composition as part of a dosage regimen, local concentrations may be allowed to reach a desired concentration for a modified FGF-1 over a series of doses.

As mentioned above, the modified FGF-1 polypeptide increases the binding affinity to heparin of wild-type human FGF-1 having SEQ ID NO: 1. This is achieved by forming a modified FGF-1 polypeptide having at least 80% amino acid sequence identity to SEQ ID NO: 1 where the serine at an amino acid position of the modified FGF-1 polypeptide corresponding to amino acid position 116 of SEQ ID NO: 1 is an amino acid that increases the modified FGF-1 polypeptide's binding affinity for heparin relative to the binding affinity of SEQ ID NO: 1 to heparin.

The modified FGF-1 polypeptide can be formed using conventional protein synthesis techniques such as liquid-phase synthesis, solid-phase synthesis, and/or by recombinant expression and purification. The expression and purification technique involves obtaining artificial genes with the desired nucleic acid sequence for expressing the synthetic polypeptide with the desired polypeptide sequence. Expression of the synthetic protein may be performed by bacteria cells. The cells are subsequently lysed and the synthetic protein is purified from the lysed cells.

The modified FGF-1 may be used to treat a physiological condition associated with insufficient blood flow. An example of a method of treating a physiological condition associated with insufficient blood flow includes administering to a subject in need thereof a composition comprising a therapeutically effective amount of a modified FGF-1 polypeptide having at least 80% amino acid sequence identity to wild-type human FGF-1 having SEQ ID NO: 1. The serine at an amino acid position of the modified FGF-1 polypeptide corresponding to amino acid position 116 of SEQ ID NO: 1 is substituted by an amino acid that increases the modified FGF-1 polypeptide's binding affinity for heparin relative to the binding affinity of SEQ ID NO: 1 to heparin.

The physiological condition associated with insufficient blood flow may be, for example, ischemia, hypoxia, peripheral artery disease, coronary artery disease, skin lesions, wounds, vascular occlusion, vascular disease, and/or avascular necrosis.

The subject may be a human or animal subject.

The pharmaceutical composition may be administered via a number of techniques. The selection of the administration technique depends on the specific area to be treated and whether local or systemic treatment is preferred. Administration techniques include, for example, enteral, parenteral, topical, or local administration techniques.

Enteral routes for administration include, for example, oral, rectal, intestinal, and gastric routes.

Parenteral routes for administration include, for example, intravenous, transmucosal, inhalation, intracranial intraocular, intrathecal, intraperitoneal, subcutaneous, intramuscular, and intradermal routes.

Some administration techniques involve administering the pharmaceutical composition as unit dosage form. For example, unit dosage forms suitable for oral administration include solid dosage forms, such as powders, granules, tablets, pills, capsules, suppositories, and sachets. They also include liquid dosage forms, such as elixirs, syrups, suspensions, sprays, gels, lotions, creams, foams, ointments, salves, solutions, tinctures, and emulsions.

The pharmaceutical composition may be administered either locally or systemically. Examples of local delivery routes include, for example, parenteral administration, local injection, or topical application at or near a site of the body to be treated. That site may be associated with a disorder or disease, such as a site afflicted with ischemia or a site of an injury or wound.

A pharmaceutical composition for parenteral administration may be formulated as a solution, emulsion, suspension, or other liquid, such as saline, dextrose solution, glycerol, and the like.

The pharmaceutical composition may be administered by local injection, placement, catheter, or implantation at the desired site of in the body of the treatment subject.

The pharmaceutical composition may be administered topically, such as at a site of a tissue injury or a wound.

The topical administration technique may include a polymer matrix that controls the release of the modified FGF-1 polypeptide at the site of administration.

Cells may be grown outside the body for use in transplantation, to a treatment subject. Culturing cells *ex vivo* may allow for their expansion and/or manipulation prior to use. A modified FGF-1 polypeptide may be introduced to the cell culture to improve the proliferation of the cells.

### EXAMPLES

This section provides specific examples of the composition and method aspects. The scope of the possible aspects and embodiments is not limited to what these examples teach.

### Example 1

### Modified FGF-1 Polypeptide Heparin Binding Affinity

### Mutagenesis and purification

A codon-optimized synthetic gene encoding 140 amino acids form of human FGF-1 (SEQ ID NO: 1) with an N-terminal six His tag was cloned into the pET21a (+) expression vector. The His tag has shown no influence upon stability, heparin affinity and mitogenic activity. Residue Ser116 inside of the heparin binding site of FGF-1 was mutated to Arginine (SEQ ID NO: 2) and Lysine (SEQ ID NO: 3) individually to increase potential ionic interactions with the sulfonate groups in HSGAG. This was achieved using a QUICKCHANGE site-directed mutagenesis protocol.

Each mutation was confirmed by DNA sequencing. The pET21a (+)/BL21(DE3) E. coli host expression system was used to express the FGF-1 heparin affinity mutants. The expressed polypeptide was purified via sequential nickel-nitrilotriacetic acid (Ni-NTA) column (Qiagen) and heparin Sepharose resin (GE Life Sciences, Pittsburgh PA). Previous studies of wild-type FGF-1 have shown that the above purification procedure results in protein >99% pure and appropriate for all biophysical studies.

### Heparin affinity study with isothermal titration calorimetry (ITC)

Purified modified FGF-1 samples of SEQ ID NOs: 2 and 3 were dialyzed into 20 mM N-(2-acetamido) iminodiacetic acid (ADA), 100 mM NaCl, pH 7.4 for an ITC study. An iTC200 microcalorimeter (MicroCal) was used for data collection. Each modified FGF-1 sample had a polypeptide concentration of 60 µM and was titrated with 750 µM sucrose octasulfate. The ITC running parameters were: 31 injections; first injection volume 0.5 µl and the rest of the injections 1µl; temperature 25°C; reference power 5. The titration curve after baseline subtraction was fitted with a single ligand-binding site model by manufacturer's software (MicroCal Origin). At least three independent trials were collected for each modified FGF-1 samples.

### Results

**Table 1** shows the quantification of the affinity between FGF-1 wild-type and the modified FGF-1 samples and sucrose octasulfate (HSGAG analog) by ITC.

**Table 1. ITC Results**

| **Peptide** | **N (site)¹** | **K_{d} (µM) ²** | **ΔH (cal/mol)** | **ΔS (cal/mol/deg)** | **Temp. (°C)** |
|---|---|---|---|---|---|
| hisFGF wild-type | 0.917±0.050 | 3.94±0.19 | (-7506)±95 | (-0.44)±0.23 | 25 |
| no histag FGF wild-type | 0.980±0.035 | 3.85±0.09 | (-6963)±65 | 1.42±0.18 | 25 |
| hisFGF S116R | 0.942±0.036 | 2.46±0.25 | (-7081)±217 | 1.84±0.92 | 25 |
| hisFGF S116K | 1.012±0.014 | 3.10±0.47 | (-3339)±57 | (-6.42)±3.04 | 25 |

| | | | | | |
|---|---|---|---|---|---|
| ¹ The titration curves were fitted using the manufacture's software (MicroCal Origin) with a single ligand-binding site model ² K_{d} is dissociation constant. The smaller value indicates the higher affinity. | | | | | |

### Conclusion

The single mutation Ser116Arg or Ser116Lys in FGF-1 increased the affinity of FGF-1 towards HSGAG.

Based on the previous pharmacokinetic study, the FGF-1 binding affinity to heparin/HSGAG is a key determinant of the distribution and elimination kinetics in the pharmacokinetic profile. Therefore, the Ser116Arg or Ser116Lys mutants may have an altered pharmacokinetic profile: a shorter distribution half-life, a longer elimination half-life and a longer mean residence time, relative to wild-type FGF-1.

The changes in pharmacokinetic profile of the Ser116Arg and Ser116Lys mutants may result in increased therapeutic efficacy by prolonging the duration of the modified FGF-1 polypeptides inside the body.

### Example 2

### Mitogenic Assay Studies

### BaF3/FGFR-1c cell culture and mitogenic stimulation by FGF-1 and S116R mutant protein

A BaF3 cell system expressing FGFR-1c was used to quantify receptor activation by wild-type FGF-1. The BaF3 cells lack membrane-bound HS proteoglycan. Heparan sulfate was added to the assay to promote the ternary FGF/FGFR/HS signal transduction complex. Cells were maintained in RPMI 1640 media (Sigma Chemical, St. Louis MO) supplemented with 10 % newborn bovine serum (Sigma Chemical, St. Louis MO), 0.5 ng/mL murine recombinant interleukin-3 (mIL-3, PeproTech Inc, Rocky Hill NJ), 2 mM L-glutamine, penicillin-streptomycin and 50 µM β-mercaptoethanol ("BaF3 culture medium"), and G418 (600 µg/mL). FGFR-1c expressing BaF3 cells were washed twice in BaF3 "assay media" ("culture media" lacking mIL-3) and plated at a density of 30,000 cells per well in a 96-well assay plate in assay media containing heparin (1 µg/mL). Concentrations of recombinant wild-type FGF-1 and modified FGF-1 (S116R mutant protein) ranged from 0.02 to 5 nM (3.18x10² - 7.95x10⁴ pg/mL). The cells were incubated for 36 hours and mitogenic activity was determined by adding 1 µCi of ³H-thymidine in 50 µl of BaF3 assay medium to each well. Cells were harvested after 4 hours by filtration through glass fiber paper. Incorporated ³H-thymidine was counted on a Wallac β plate scintillation counter (PerkinElmer, Waltham MA).

### 3T3 Fibroblast mitogenic stimulation by FGF-1 and S116R mutant protein

The mitogenic activity of wild-type FGF-1 and S116R towards NIH 3T3 fibroblasts was studied. In contrast to BaF3 cells, NIH 3T3 fibroblast cells do express HS proteoglycan, thus, no heparan sulfate was added in this assay.

NIH 3T3 fibroblasts were initially plated in Dulbecco's modified Eagle's medium (DMEM) (American Type Culture Collection, Manassas VA) supplemented with 10% (v/v) newborn calf serum (NCS) (Sigma, St Louis MO), 100 units of penicillin, 100 mg of streptomycin, 0.25 mg of Fungizone and 0.01 mg/mL of gentamicin (Gibco, Carlsbad CA) (serum-rich medium) in T75 tissue culture flasks (Fisher, Pittsburgh PA). The cultures were incubated at 37 °C with 5% (v/v) CO₂ supplementation. At ∼80% cell confluence, the cells were washed with 5 mL of TBS (0.14 M NaCl, 5.1 mM KCl, 0.7 mM Na₂HPO₄, 24.8 mM Trizma base (pH 7.4)) and subsequently treated with 5 mL of a 0.025% (v/v) trypsin solution (Invitrogen Corp., Carlsbad CA).

The trypsinized cells were then seeded in T25 tissue culture flasks at a density of 3×10⁴ cells/cm² (representing ∼20% confluence). Cell quiescence was initiated by serum starvation in DMEM with 0.5% NCS, 100 units of penicillin, 100 mg of streptomycin, 0.25 mg of Fungizone and 0.01 mg/mL of gentamicin. Cultures were incubated for 48 hours at 37°C, the medium was then decanted and replaced with fresh medium supplemented with wild-type or S116R FGF-1 protein from 0.063 to 630 nM (1x10³ - 1x10⁷ pg/mL), and the cultures incubated for an additional 48 hours.

After this incubation, the medium was decanted and the cells were washed with 1 mL of TBS. Then 1 mL of 0.025% trypsin was then added to release the cells from the flask surface, and 2 mL of serum-rich medium was added to dilute and inhibit the trypsin. Triplicate cell samples were counted using a Coulter Counter (Beckman Coulter, Brea CA). Dose response experiments were performed in quadruplicate, and the cell counts were averaged and normalized to a % maximal stimulation in reference to 10% NCS stimulation.

### Results

The mitogenic response of BaF3 cells expressing FGFR-1c towards wild-type FGF-1 and S116R mutant (in the presence of added heparin in the assay) is shown in **FIG**. **5A**. The assay was able to quantify the mitogenic response of WT FGF-1 over a concentration range of ∼2.5-5 log pg/mL. Under these conditions the mitogenic response of wild-type FGF-1 as a function of concentration did not achieve saturation conditions, while the S116R mitogenic activity achieved approximate saturation.

The S116R mutant exhibited an increase in mitogenic activity in comparison to wild-type FGF-1 over the range of approximately 3-4 log pg/mL. The S116R mutant exhibited about 10 times greater mitogenic potency, on an equivalent concentration basis, compared to wild type FGF-1.

The mitogenic activity of WT FGF-1 and S116R towards NIH 3T3 fibroblasts (in the absence of exogenously added heparin in the assay) is shown in **FIG**. **5B**. NIH 3T3 cells appeared less responsive to FGF-1 than the BaF3/FGFR-1c cells, and a mitogenic response was quantified primarily over a concentration range of 5-7 log pg/mL. Over this range neither wild-type FGF-1 nor S116R achieves a saturation condition. The S116R mutant exhibited an increase in mitogenic activity in comparison to WT FGF-1. Over the range of approximately 5-7 log pg/mL, the S116R mutant exhibited about ten times greater mitogenic potency, on an equivalent concentration basis, compared to wild-type FGF-1.

### Discussion

The BaF3/FGFR-1c and NIH 3T3 assays of mitogenic activity indicated an about 10-fold increase in mitogenic activity for S116R, identifying S116R as a gain-of-function mutation.

This disclosure describes preferred embodiments, but not all possible embodiments of the compositions and methods. Where a particular feature is disclosed in the context of a particular composition or method, that feature can also be used, to the extent possible, in combination with and/or in the context of other embodiments of the compositions and methods. The compositions and methods may, be embodied in many different forms and should not be construed as limited to only the embodiments described here.

## Claims

1. A modified FGF-1 polypeptide having at least 80% amino acid sequence identity to wild-type human FGF-1 having SEQ ID NO: 1, the serine at an amino acid position of the modified FGF-1 polypeptide corresponding to amino acid position 116 of SEQ ID NO: 1 being substituted by an arginine, and wherein the arginine increases the modified FGF-1 polypeptide's binding affinity for heparin relative to the binding affinity of SEQ ID NO:1 to heparin.

2. The modified polypeptide of claim 1, wherein the modified FGF-1 polypeptide has at least 90% amino acid sequence identity to wild-type human FGF-1 having SEQ ID NO: 1.

3. The modified polypeptide of claim 2, wherein the modified FGF-1 polypeptide has at least 99 % amino acid sequence identity to wild-type human FGF-1 having SEQ ID NO: 1.

4. The modified polypeptide of any one of claims 1 to 3, wherein the modified FGF-1 polypeptide comprises or consists of SEQ ID NO: 2.

5. A composition comprising the modified FGF-1 polypeptide as defined in any one of the preceding claims.

6. A pharmaceutical composition comprising the modified FGF-1 polypeptide as defined in any one of the preceding claims and one or more pharmaceutically acceptable excipients.

7. A method of increasing the binding affinity to heparin of wild-type human FGF-1 having SEQ ID NO: 1, the method comprising forming a modified FGF-1 polypeptide as defined in any one of claims 1 to 4.

8. The modified polypeptide of any one of claims 1 to 4, or the composition of claims 5 or 6, for use in therapy, medicine or as a medicament.

9. The modified polypeptide of any one of claims 1 to 4, or the composition of claims 5 or 6, for use in a method of treating peripheral artery disease or skin lesions.

10. The modified polypeptide of any one of claims 1 to 4, or composition of claims 5 or 6, for use in a method of treating ischemia, hypoxia or avascular necrosis.

11. The modified polypeptide of any one of claims 1 to 4, or the composition of claims 5 or 6 for use in a method of treating a wound in a human or animal subject.

12. The modified polypeptide or composition for use according to claim 11, wherein the subject is a human subject.

## Patentansprüche

1. Modifiziertes FGF-1-Polypeptid mit mindestens 80 % Aminosäureidentität zu menschlichem Wildtyp-FGF-1 mit SEQ ID NO: 1, wobei das Serin an einer Aminosäureposition des modifizierten FGF-1-Polypeptids, die Aminosäureposition 116 von SEQ ID NO: 1 entspricht, durch ein Arginin substituiert wird und wobei das Arginin die Bindungsaffinität des modifizierten FGF-1-Polypeptids für Heparin relativ zur Bindungsaffinität von SEQ ID NO: 1 an Heparin erhöht.

2. Modifiziertes Polypeptid nach Anspruch 1, wobei das modifizierte FGF-1-Polypeptid mindestens 90 % Aminosäuresequenzidentität zu menschlichem Wildtyp-FGF-1 mit SEQ ID NO: 1 aufweist.

3. Modifiziertes Polypeptid nach Anspruch 2, wobei das modifizierte FGF-1-Polypeptid mindestens 99 % Aminosäuresequenzidentität zu menschlichem Wildtyp-FGF-1 mit SEQ ID NO: 1 aufweist.

4. Modifiziertes Polypeptid nach einem der Ansprüche 1 bis 3, wobei das modifizierte FGF-1-Polypeptid SEQ ID NO: 2 umfasst oder daraus besteht.

5. Zusammensetzung, umfassend das modifizierte FGF-1-Polypeptid wie definiert nach einem der vorhergehenden Ansprüche.

6. Pharmazeutische Zusammensetzung, umfassend das modifizierte FGF-1-Polypeptid wie definiert nach einem der vorhergehenden Ansprüche und ein oder mehrere pharmazeutisch annehmbare Hilfsstoffe.

7. Verfahren eines Erhöhens der Bindungsaffinität an Heparin von menschlichem Wildtyp-FGF-1 mit SEQ ID NO: 1, wobei das Verfahren ein Bilden eines modifizierten FGF-1-Polypeptids wie definiert nach einem der Ansprüche 1 bis 4 umfasst.

8. Modifiziertes Polypeptid nach einem der Ansprüche 1 bis 4 oder Zusammensetzung nach Anspruch 5 oder 6 zur Verwendung in der Therapie, Medizin oder als ein Medikament.

9. Modifiziertes Polypeptid nach einem der Ansprüche 1 bis 4 oder Zusammensetzung nach Anspruch 5 oder 6 zur Verwendung in einem Verfahren eines Behandelns von peripherer Arterienerkrankung oder Hautläsionen.

10. Modifiziertes Polypeptid nach einem der Ansprüche 1 bis 4 oder Zusammensetzung nach Anspruch 5 oder 6 zur Verwendung in einem Verfahren eines Behandelns von Ischämie, Hypoxie oder avaskulärer Nekrose.

11. Modifiziertes Polypeptid nach einem der Ansprüche 1 bis 4 oder Zusammensetzung nach Anspruch 5 oder 6 zur Verwendung in einem Verfahren eines Behandelns einer Wunde bei einem menschlichen oder tierischen Subjekt.

12. Modifiziertes Polypeptid oder Zusammensetzung zur Verwendung nach Anspruch 11, wobei das Subjekt ein menschliches Subjekt ist.

## Revendications

1. Polypeptide de FGF-1 modifié présentant au moins 80 % d'identité de séquence d'acide aminé au FGF-1 humain sauvage comportant la SEQ ID n° : 1, la sérine dans une position d'acide aminé du polypeptide de FGF-1 modifié correspondant à la position d'acide aminé 116 de la SEQ ID n° : 1 étant substituée par une arginine, et l'arginine augmentant l'affinité de liaison du polypeptide de FGF-1 modifié pour l'héparine par rapport à l'affinité de liaison de la SEQ ID n° : 1 à l'héparine.

2. Polypeptide modifié selon la revendication 1, le polypeptide de FGF-1 modifié présentant au moins 90% d'identité de séquence d'acide aminé au FGF-1 humain sauvage comportant la SEQ ID n° : 1.

3. Polypeptide modifié selon la revendication 2, le polypeptide de FGF-1 modifié présentant au moins 99 % d'identité de séquence d'acide aminé au FGF-1 humain sauvage comportant la SEQ ID n° : 1.

4. Polypeptide modifié selon l'une quelconque des revendications 1 à 3, dans lequel le polypeptide de FGF- 1 modifié comprend la SEQ ID n° : 2 ou est constitué de celle-ci.

5. Composition comprenant le polypeptide de FGF-1 modifié tel que défini dans l'une quelconque des revendications précédentes.

6. Composition pharmaceutique comprenant le polypeptide de FGF-1 modifié tel que défini dans l'une quelconque des revendications précédentes et au moins un excipient pharmaceutiquement acceptable.

7. Procédé d'augmentation de l'affinité de liaison à l'héparine ou au FGF-1 humain sauvage comportant la SEQ ID n° : 1, le procédé comprenant la formation d'un polypeptide de FGF-1 modifié tel que défini dans l'une quelconque des revendications 1 à 4.

8. Polypeptide modifié selon l'une quelconque des revendications 1 à 4, ou composition selon les revendications 5 ou 6, en vue d'une utilisation dans un traitement, une préparation médicinale ou un médicament.

9. Polypeptide modifié selon l'une quelconque des revendications 1 à 4, ou composition selon les revendications 5 ou 6, en vue d'une utilisation dans une méthode de traitement d'une maladie artérielle périphérique ou de lésions de la peau.

10. Polypeptide modifié selon l'une quelconque des revendications 1 à 4, ou composition selon les revendications 5 ou 6, en vue d'une utilisation dans une méthode de traitement de l'ischémie, l'hypoxie ou la nécrose avasculaire.

11. Polypeptide modifié selon l'une quelconque des revendications 1 à 4, ou composition selon les revendications 5 ou 6, en vue d'une utilisation dans une méthode de traitement d'une blessure chez un sujet humain ou animal.

12. Polypeptide modifié ou composition en vue d'une utilisation selon la revendication 11, dans lequel le sujet est un sujet humain.
